# EUROPEAN PATENT SPECIFICATION

(11) **EP 1 726 319 B1**
(45) Date of publication and mention of the grant of the patent: **08.10.2008**
(21) Application number: 05104516.9
(22) Date of filing: 26.05.2005
(51) Int. Cl.: A61L 27/30, A61L 27/32, A61L 27/50

(54) **Implant device with a coating comprising cavities**
Implantatvorrichtung mit Hohlräume enthaltende Beschichtung
Dispositif d'implant avec des cavités dans le revêtement

(43) Date of publication of application: 29.11.2006
(73) Proprietor: Doxa AB, 754 51 Uppsala (SE)
(72) Inventor: HERMANSSON, Leif, 260 42, Mölle (SE)
(74) Representative: Holmberg, Nils Anders Patrik

(56) References cited:
- EP-A- 0 559 508
- WO-A-03/074097
- WO-A-20/04040036
- DE-A1- 19 830 530
- US-A- 5 480 438
- US-A1- 2002 076 528

## Description

### TECHNICAL FIELD

The present invention generally relates to dental and orthopaedic implants, and more specifically to an implant coated with a chemically bonded ceramic, having a modified substrate surface to improve adhesion of the implant coating to the implant material, wherein the surface of the implant is provided with a specific geometry or topology filled with the coating material improving the anchoring of the coating to the implant surface and providing for tissue ingrowth.

### BACKGROUND ART

For implants that are to interact with the human body, implant materials that due to their biocompatibility provide an optimal fixation or anchoring of the implant to the biological tissue, e.g. bone, are advantageous. To allow for early loading of an implant and to reduce the risk for long term loosening, high quality fixation is important. In the case of a coated implant, the anchoring of the coating to the implant surface is often the weak spot of the implant system. The adhesion strength of the coating to the implant is often too low, and stress is often found in the contact zone due to mismatch in properties of the implant material, on the one hand, and the coating material, on the other hand.

The contact zone of an implant to the living tissue is critical and often controls the survival (the life time) of an implant. The degree of bone ingrowth towards the implant, the rate of ingrowth, and possible integration of tissue with implant control the behaviour of the contact zone. By coating especially metallic implant surfaces with a layer of chemically bonded ceramics, the growth and the chemical integration of bone and implant can be improved. Such examples include plasma-sprayed hydroxyapatite (10-100 µm thick layers) and physical vapour deposited (PVD) hydroxyapatite (0.1-10 µm thick layer). A problem which has reduced the use of these coatings is the low adhesion of the coating to the implant.

Accordingly, it is an object of the present invention to provide a coated implant exhibiting improved adhesion of the coating to the surface of the implant material. Also, the implant system should fulfill the requirements on implantation system and materials, such as desired porosity and desired thickness to optimize the mechanical property profile, i.e. high shear strength of the layer towards the substrate and reduced thickness of the layer to eliminate larger defects in the layer/coating.

For an implant having a coating of a chemically bonded ceramic material this object has been achieved by means of the implant surface underlying the coating exhibiting a specified topology as defined in claim 1.

### SUMMARY OF THE INVENTION

Accordingly, in a first aspect the invention relates to an implant having a coating of a chemically bonded ceramic material with improved adhesion to the underlying implant surface, wherein the surface exhibits a specified surface microstructure containing cavities having a width "a", a depth "c", and a distance between neighbouring cavities "b" of 80<a<200; 50<b<400; and 20<c<200 µm. The cavities are filled with the CBC material. The adhesion is improved by increased mechanical interlocking and the shear strength is equal to that of the coating layer. After implantation the filled cavities are successively replaced by new tissue, since the chemically bonded material used are of slowly resorbable or resorbable chemistry.

In another aspect the invention relates to a method of preparing the implant of the invention.

In a further aspect the invention relates to a method of implanting the implant of the invention into a mammal in need thereof, e.g. for veterinary purpose.

Further advantages and embodiments will be evident from the detailed description and appended claims.

### BRIEF DESCRIPTION OF THE DRAWING

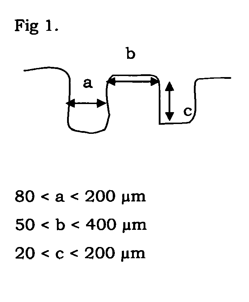

Figure 1 shows the surface structure of a surface of an implant device to be coated,

### i.e. to be filled with the coating material.

### DETAILED DESCRIPTION OF THE INVENTION

The present invention relates to the anchoring of implant systems with binding agent systems of the hydrating cement type as coating material, in particular of aluminates, silicates, phosphates, fluorides, carbonates, sulphates and combinations thereof, preferably having calcium as the major cation. This in combination with a pre-treated implant surface according to the present invention yields implant fixation systems of high quality. The invention is especially intended for dental and orthopaedic implants for load bearing applications and for carrier for drugs and/or biosensors. The implant may be metallic, polymeric or ceramic.

Examples of suitable materials for use in the present invention are those described in SE 463 493, SE 502 987, SE 514 686, SE 516 264, SE 524 494, SE 521 938 and SE 524 334, SE 521 973, SE 522 749, 522 749, PCT/SE 2003/001679 and PCT/SE 2004/001745,

As mentioned above, one problem which has reduced the use of these coatings is the low adhesion of the coating to the implant. The present inventors believe the low adhesion to be due to the different chemical natures of the implant and the coating material, respectively, as well as mechanical stress developed during processing due to mismatch in physical properties, e.g. in the thermal expansion coefficient. Low shear strength in the contact zone between coating and underlying implant surface has increased the risk of a fall off of the coating *in vivo* after some time, and consequently a loss of the benefit of the coating, and a possible concomitant inflammation.

The present invention aims at providing an implant having a coating based on chemically bonded ceramic materials (CBC-materials) for *in vivo* anchoring of the implant to a biological tissue, wherein the adhesion aspects of the coating to the bulk implant material is of prime interest.

According to the invention, in order to increase the adhesion of the coating to the implant, a structured surface of the implant material is used containing cavities as shown in Figure 1, wherein the un-hydrated or partially hydrated chemically bonded ceramics used fill the cavities in the implant surface. The cavities are typically 100 µm in depth (c), 100 µm in width (a) and the distance (b) between the cavities is approximately 100 µm, but could vary within the intervals 80<a<200 µm, 50<b<400 µm, and 20<c<200 µm. Methods for forming the topography include laser and ultrasonic drilling. Accordingly, portions of the implant surface where improved adhesion of the invention is desired must be provided with the above-mentioned topography. The cavities are afterwards filled with the CBC material in the unhydrated, partially hydrated or fully hydrated state.

The final hydration occurs *in vivo* in contact with body liquid. This reaction involves a mass increase meaning that also a part of the distance between the cavities will be covered by the chemically bonded ceramic. The system with filled cavities of the above defined size increases the adhesion strength to bone initially - within a few hours. See Example 1. The distance between the cavities may be pre-treated to provide for an inter-distance coating with preferably a thin coating (< 10 µm) of the same type of material as used in the cavities.

In the case with resorbable material, such as chemically bonded ceramics of silicate and sulphate type, the filled cavities will after some time be replaced with new formed bone tissue if the cavity size exceeds approximately 100 µm. Accordingly, the chemically bonded ceramic used should preferably be resorbable or slowly resorbable.

In its most generic embodiment the method of preparing the implant system involves the following steps:
a) Providing an implant device;
c) Applying a coating of a chemically bonded material to a surface of the implant device; wherein step c) is preceded by the following step:
b) Forming on said surface a surface structure containing cavities having a width "a", a depth "c", and a distance between neighbouring cavities "b", wherein 80<a<200; 50<b<400; and 20<c<200 µm.

In step c) the coating is preferably applied so as to essentially fill the cavities. The filling of the cavities can be done in several ways including slip casting, dry pressing (conventional or cold isostatic pressing) or by a paste smeared/pressed into the cavities, and left in the non-hydrated or hydrated state.

In a subsequent step d), preferably immediately before implantation, partial or complete hydration of the reactive coating material can be carried out.

Thereafter, in a step e), the implantation takes place. The CBC-material will then be anchored to both the implant material and the biological tissue by dissolution-precipitation and volume increase and by bioactive bonding to the surrounding tissue.

The present invention will now be illustrated by means examples which are provided for the purpose of illustration only and should not be construed as limiting the invention except as defined by the claims.

### EXAMPLES

### Example 1

The implant material used was cp Ti in the form of cylinders (length, 1 =5 mm and diameter, φ = 4 mm). By using laser drilling a topography according to Figure 1 was obtained, where a, b and c varied according to Table 1.

**Table 1: Distances a, b and c in the tests performed.**

| Test No | a in µm | b in µm | c in µm |
|---|---|---|---|
| 1 | 100 | 100 | 100 |
| 2 | 50 | 100 | 50 |
| 3 | 150 | 100 | 150 |
| 4 | 200 | 200 | 200 |
| 5 | - | - | - |

In the tests 1-4 according to Table 1 above Ca-aluminate (CA with a CAO:Al₂O₃ ratio of 1:1) powder was pressed into the cavities with a pressure of 20 MPa. This yielded a green density of 42 %. The material was dipped into a phosphate buffer system (pH 7.2) for 10 s and thereafter immediately implanted into the femur of New Zeeland white rabbits (5 kg) for 2 weeks, 6 weeks, 3 months and 6 months. The powder mix was sterilized by electron beam radiation, and the aqueous solutions were steam sterilized. The shear strength was measured with push-out testing in a universal testing machine (Zwick). The results are shown in Table 2 below.

**Table 2**

| Material | Shear strength MPa, 2 weeks | Shear strength MPa, 6 weeks | Shear strength MPa, 3 months | Shear strength MPa, 6 months |
|---|---|---|---|---|
| 1 | 6 | 8 | 12 | 12 |
| 2 | 6 | 8 | 12 | 12 |
| 3 | 6 | 8 | 12 | 12 |
| 4 | 4 | 7 | 9 | 9 |
| 5 | 0.5 | 2 | 3 | 5 |

### Example 2

In the tests 1-4 Ca-silicate (C₃S with a CaO:SiO₂ ratio of 3:1) powder was pressed into the cavities with a pressure of 20 MPa. This yielded a green density of 40 %. The material was dipped into a phosphate buffer system (pH 7.2) for 10 s and thereafter implanted into the femur of New Zeeland white rabbits (5 kg) for 6h, 2 weeks, 6 weeks, and 12 weeks. The powder mix was sterilized by electron beam radiation, and the aqueous solutions were steam sterilized. The ingrowth length into cavity from the surface was measured.

| Material | Ingrowth length in µm 6 h | Ingrowth length in µm 2 weeks | Ingrowth length in µm 6 weeks | Ingrowth length in µm 12 weeks |
|---|---|---|---|---|
| 1 | - | 25 | 50 | 80 |
| 2 | - | - | - | - |
| 3 | - | 25 | 50 | 110 |
| 4 | - | Approx. 25 | Approx. 50 but uneven | Approx. 100 but uneven |

## Claims

1. Implant device having a surface coated with a chemically bonded ceramic material having improved adhesion of the coating to the surface, **characterized in that** the surface exhibits a structure containing filled cavities having a width "a", a depth "c", and a distance between neighbouring cavities "b", wherein 100<a<200; 50<b<400; and 20<c<200 µm.

2. The implant device of claim 1, wherein the bonded ceramic material is selected from aluminates, silicates, phosphates, fluorides, carbonates and/or sulphates with Ca as dominating cation.

3. The implant device of claim 1 or 2, wherein the bonded ceramic material is resorbable or slowly resorbable.

4. The implant device of any of the previous claims, wherein the implant material is based on Ti, Ta, Co-Cr or stainless steel.

5. Method of preparing an implant of claim 1, including the following steps:
providing an implant device;
applying a coating of a chemically bonded ceramic material to a surface of the implant device;
**characterized in** also including the following step:
before coating thereof forming on said surface of the implant device a structure containing cavities having a width "a", a depth "c", and a distance between neighbouring cavities "b", wherein 100<a<200; 50<b<400; and 20<c<200 µm.

6. The method of claim 5, wherein the coating is applied so as to fill the cavities.

## Patentansprüche

1. Implantatvorrichtung mit einer Oberfläche, welche mit einem chemisch gebundenem keramischen Material beschichtet ist, welches eine verbesserte Adhäsion der Beschichtung an die Oberfläche hat, **dadurch gekennzeichnet, dass** die Oberfläche eine Struktur enthaltend gefüllte Hohlräume mit einer Breite 'a', einer Tiefe 'c' und einem Abstand zwischen benachbarten Hohlräumen 'b' besitzt, wobei 100<a<200; 50<b<400; und 20<c<200µm.

2. Implantatvorrichtung nach Anspruch 1, wobei das gebundene keramische Material ausgewählt ist aus Aluminaten, Silikaten, Phosphaten, Fluoriden, Carbonaten und/oder Sulphaten mit Ca als vorherrschendes Kation.

3. Implantatvorrichtung nach Anspruch 1 oder 2, wobei das gebundene keramische Material resorbierbar oder langsam resorbierbar ist.

4. Implantatvorrichtung nach einem der vorangehenden Ansprüche, wobei das implantierbare Material auf Ti, Ta, Co-Cr oder Edelstahl basiert.

5. Verfahren zur Herstellung eines Implantats nach Anspruch 1, einschließlich der folgenden Schritte:
Bereitstellen einer lmplantatvorrichtung;
Aufbringen einer Beschichtung eines chemisch gebundenen keramischen Materials auf die Oberfläche der lmplantatvorrichtung;
**dadurch gekennzeichnet, dass** auch der folgende Schritt eingeschlossen ist:
Bilden einer Struktur auf der Oberfläche der lmplantatvorrichtung vor deren Beschichtung, enthaltend Hohlräume mit einer Breite 'a', einer Tiefe 'c' und einem Abstand zwischen benachbarten Hohlräumen 'b', wobei 100<a<200; 50<b<400; und 20<c<200µm.

6. Verfahren nach Anspruch 5, wobei die Beschichtung so angewendet wird dass die Hohlräume gefüllt werden.

## Revendications

1. Dispositif d'implant ayant une surface revêtue avec un matériau céramique lié chimiquement ayant une adhésion améliorée du revêtement à la surface, **caractérisé en ce que** la surface présente une structure contenant des cavités remplies ayant une largeur "a", une profondeur "c" et une distance entre des cavités voisines "b", où 100<a<200 ; 50<b<100 ; et 20<c<200 µm.

2. Dispositif d'implant selon la revendication 1, dans lequel le matériau céramique lié est choisi parmi les aluminates, les silicates, les phosphates, les fluorures, les carbonates et/ou les sulfates avec Ca en tant que cation dominant.

3. Dispositif d'implant selon la revendication 1 ou 2, dans lequel le matériau céramique lié est résorbable ou lentement résorbable.

4. Dispositif d'implant selon l'une quelconque des revendications précédentes, dans lequel le matériau d'implant est à base de Ti, Ta, Co-Cr ou d'acier inoxydable.

5. Procédé de préparation d'un implant selon la revendication 1, incluant les étapes suivantes :
fourniture d'un dispositif d'implant ;
application d'un revêtement d'un matériau céramique lié chimiquement à une surface du dispositif d'implant ;
**caractérisé en ce qu'**il inclut aussi l'étape suivante:
avant le revêtement de celui-ci, la formation sur ladite surface du dispositif d'implant d'une structure contenant des cavités ayant une largeur "a", une profondeur "c" et une distance entre des cavités voisines "b", où 100<a<200 ; 50<b<400 ; et 20<c<200 µm.

6. Procédé selon la revendication 5, dans lequel le revêtement est appliqué de façon à remplir les cavités.
